## Europäisches Patentamt
## European Patent Office
## Office européen des brevets

(19)

(11) Publication number: **0 039 723**
**B1**

(12) **EUROPEAN PATENT SPECIFICATION**

(45) Date of publication of patent specification: **11.04.84**

(21) Application number: **80902320.3**

(22) Date of filing: **14.10.80**

(86) International application number:
**PCT/US80/01375**

(87) International publication number:
**WO 81/01284 14.05.81 Gazette 81/12**

(51) Int. Cl.³: **C 07 C 17/02,**
**B 01 J 27/06, C 01 B 7/00**

(54) IMPROVED ALUMINA-SUPPORTED COPPER CATALYST COMPOSITIONS FOR FLUID-BED HYDROCARBON OXYHYDROCHLORINATION.

(30) Priority: **05.11.79 US 91288**

(43) Date of publication of application:
**18.11.81 Bulletin 81/46**

(45) Publication of the grant of the patent:
**11.04.84 Bulletin 84/15**

(84) Designated Contracting States:
**DE FR GB NL SE**

(56) References cited:
**CA - A - 695 895**
**CA - A - 701 913**
**US - A - 2 271 056**
**US - A - 3 427 359**
**US - A - 3 461 181**
**US - A - 3 488 398**
**US - A - 3 642 921**
**US - A - 3 709 950**
**US - A - 4 069 170**
**US - A - 4 124 534**
**US - A - 4 206 180**

(73) Proprietor: **The B.F. GOODRICH Company**
**Dept. 0015 WHB-6 500 South Main Street**
**Akron, Ohio 44318 (US)**

(72) Inventor: **EDEN, Jamal Shahab**
**502 North Revere Road**
**Akron, OH 44313 (US)**

(74) Representative: **von Kreisler, Alek, Dipl.-Chem. et al,**
**Deichmannhaus am Hauptbahnhof**
**D-5000 Köln 1 (DE)**

Courier Press, Leamington Spa, England.

Improved alumina-supported copper catalyst compositions for fluid-bed
hydrocarbon oxyhydrochlorination

Background of the invention

This invention pertains to fluid bed catalytic oxyhydrochlorination of hydrocarbons, particularly ethylene, to produce chlorinated hydrocarbons, particularly 1,2 dichloroethane, commonly called ethylene dichloride (EDC) and relates specifically to improved alumina-supported copper catalysts and their use in such oxyhydrochlorination.

The production of chlorinated hydrocarbons by oxyhydrochlorination (sometimes called more simply "oxychlorination") of hydrocarbons containing 1 to 4 carbon atoms is well known to the art. A particularly advantageous process for oxyhydrochlorination of ethylene to produce EDC, practiced in many highly successful commercial installations through the world, involves the reactions in the vapor phase over a fluidized catalyst bed, of a mixture of ethylene, hydrogen chloride (HCl) and oxygen, or an oxygen containing gas (e.g., air), in the manner and under the conditions generally described in Harpring et al U.S. Patent 3,488,398. In commercial operation of this process the molar ratio of ethylene to oxygen to HCl is maintained in the range of about 1.0 to 1.2 moles ethylene to about 0.55 to 0.9 moles oxygen for each 2 moles of HCl; the temperature is maintained in the range of 200 to 250°C. and the pressure is normally in the range of 10 to 50 psi.

The fluidized catalyst bed heretofore used in commercial operation consists of about 2 to 10% by weight of a copper compound, preferably copper chloride, as the active catalytic ingredient, uniformly deposited on fine particles of a fluidizable alumina support. The alumina support material, which is preferably gamma alumina but can also be "Condea" alumina, on the so-called microgel alumina or other forms of "activated" alumina, resembling fine sand, is of such a nature as to be readily fluidizable without excessive catalyst loss from the reaction zone, by virtue of having the proper bulk density, resistance to attrition and a preselected ratio of particle sizes and also, in order to provide ample reaction sites of copper catalyst, has a surface area, after copper is deposited thereon, in the range of 60 to 160 square meters per gram ($m^2g$).

However, the supported catalyst functioning in the fluid bed oxyhydrochlorination of ethylene, as above described, could desirably be improved in two significant respects.

First, it would be desirable for the catalyst to effect a higher than normal EDC efficiency based on ethylene (i.e., for the ethylene reactant to be more completely converted to EDC with less being converted to carbon oxides—carbon monoxide and carbon dioxide). This is especially desirable when the ethylene oxyhydrochlorination process as described in the Harpring et al patent (which in its exemplified embodiments used air to supply oxygen) is adapted to recycle the gases normally vented to the atmosphere, in order to avoid releasing hydrocarbons and chlorinated hydrocarbons to the environment, with introduction of essentially pure oxygen rather than air with the recycle stream. This variation of the ethylene oxyhydrochlorination process is termed herein, for brevity, "oxyvent recycle" and is described in more detail in Anoto et al U.S. Patent 4,071,572 and especially in Hoechst A. G. Belgian Patent 866,157.

Secondly, the copper-on alumina fluidized catalyst exhibits during the oxyhydrochlorination reaction a pronounced tendency to develop "stickiness" and cause severe disruption of process operations. This problem and means for its partial control are described in the copending application of Joseph Allen Cowfer, Dane Edward Jablonski, Ronald Michael Kovacs and Angelo Joseph Magistro S. N. 949,170 filed November 6, 1978 and a method and composition for alleviating or eliminating stickiness in the catalyst through use of bare alumina support and deposition of copper on bare support in situ are described in the copending related application of Joseph Alan Cowfer, Jamel Shahab Eden and Angelo Joseph Magistro filed concurrently herewith.

Finally, by way of background, it has been proposed in numerous prior art patents to conduct hydrocarbon oxychlorination reactions in a fluid bed in which the fluidized catalyst consists of a mixture of copper chloride with other metal chlorides especially potassium chloride (instead of using copper alone) both deposited on a fluidizable support. For example, U.S. Patent 3,427,359 describes a catalyst composition for fluid-bed oxychlorination of hydrocarbons and partially chlorinated hydrocarbons, such as methane and methylene chloride, consisting of copper chloride, an alkali metal chloride and a rare earth metal chloride supported on an inert carrier material such as alpha alumina having a surface area no greater than 10 $m^2g$. A similar catalyst is described in US-Patent 4 124 534. The Canadian Patent CA—A—695 895 discloses an oxychlorination-catalyst consisting of an activated alumina which has been heat treated at 600 to 1200°C and has been impregnated with copper salts and additional specific metals. However, the expedient of depositing one or more other metals with copper on the alumina support described above, and using such a supported catalyst in the fluid bed ethylene oxychlorination process described above, does not materially improve ethylene efficiency to EDC and, moreover, results in significantly increased stickiness of particles in the fluid bed. Consequently, this prior art does not point to the improvement desired in either of the two aspects noted hereinabove.

Summary of the invention

It has now been discovered, unexpectedly, that the incorporation of certain specified metals in certain proportions into the alumina support material conventionally used in preparation of the hereinabove described alumina supported copper catalysts for fluid bed ethylene oxyhydrochlorination to produce EDC, prior to and independently of depositing copper catalyst on the support, results in a novel improved alumina-supported copper catalyst which when used as the fluid bed in the oxyhydrochlorination of ethylene to EDC enables attainment of significantly improved efficiency of EDC production based on ethylene and further exhibits improved fluidization properties, not developing stickiness in use to the extent of a similar catalyst bed in which the alumina support material has not been modified by having a metal as specified incorporated therein.

The instant invention thus discloses a catalyst composition comprising a copper catalyst deposited on a fluidizable gamma alumina support, having a surface area of 60 to 200 square meters per gram, in which is incorporated prior to deposit of the copper catalyst from 0.5 to 3.0% by weight of at least one added metal selected from the class consisting of potassium, lithium, rubidium, cesium, alkaline earth metals and rare earth metals or combinations thereof.

The incorporation of a mixture of an alkali metal and an alkaline earth metal into gamma alumina support, after which copper is deposited thereon, produces a supported catalyst particularly effective for oxyvent recycle oxyhydrochlorination of ethylene.

Incorporation of the specified metal or metals into the alumina support is accomplished conveniently by inpregnating the support with an aqueous solution of a water soluble salt of the metal or metals in question, drying the wetted support and then calcining at elevated temperatures for several hours to produce an intimate admixture of the finely divided fluidizable alumina with the finely divided metal or metals in question probably in the form of complex oxides, and having essentially the same physical characteristics as the fluidizable alumina before metal is incorporated therein. The proportion of added metal in the so-modified alumina support is in the range of 0.5 to about 3.0% by weight.

Deposit of copper preferably as copper chloride, on the alumina support modified by incorporation of metal as described, produces the novel fluidizable catalyst composition of this invention. Deposit of copper on the modified support is effected in any known manner either entirely before the catalyst composition is placed in an oxyhydrochlorination reactor to function as the fluid bed or at least partly in situ in the manner described in the aforementioned concurrently-filed application of Cowfer, Eden, and Magistro.

When the novel catalyst composition is used as the fluid bed in the process of oxyhydrochlorination of ethylene to EDC under normal reaction conditions, conversion of ethylene are generally above 98%, and 98% or more of the ethylene converted yields EDC (i.e., a maximum of only about 2% of the ethylene converted leaks to carbon oxide formation) so that the EDC efficiency based on ethylene (% conversion of ethylene × % yield EDC based on ethylene) is of the order of 97% or higher. This efficiency compares with an EDC efficiency based on ethylene generally in the order of 90%, and at best no more than 93—94%, when using the conventional catalyst composition—copper supported on alumina without prior incorporation of metal with the alumina. Further, the incorporation of metal in the alumina support renders the catalyst composition less "stickiness-prone" during the course of the oxyhydrochlorination reaction. Accordingly, this invention provides, in addition to a novel and improved catalyst composition, an improved fluid-bed hydrocarbon oxyhydrochlorination process characterized in that the fluid bed is composed of the novel catalyst composition.

Detailed description of the invention

The alumina support material used in preparing the modified alumina support material on which copper is deposited to form the catalyst compositions of this invention is readily available to catalyst manufacturers and is not strictly critical so long as it is of such characteristic as to be readily fluidizable. As previously mentioned, various types of finely divided fluidizable aluminas may be used, the material known as "gamma alumina" being especially suitable. The alumina material used normally has a surface area in the range of 60 to 200 $m^2g$, a bulk density in the range of 0.9 to 1.1 grams per c.c., a pore volume in the range of 0.2 to 0.5 c.c. per gram and a particle size distribution such that about 75 to 92 weight percent of the particles are below 80 microns in diameter, about 40 to 50 percent below 45 microns in diameter and about 15 to 30 percent below 30 microns in diameter with no more than 1 to 5% larger than 200 microns or more than 3 to 10% below 20 microns. Such alumina materials are relatively stable, mechanically strong and resistant to attrition so that excessive quantities are not lost from the fluid bed reaction zone when used as catalyst support in fluid bed reactions.

It is recognized that some conventional alumina support materials may inherently contain in addition to $Al_2O_3$ traces of other metal oxides such as sodium oxide. It is understood that use of such supports without modification through incorporation of added amounts of the metals herein specified forms no part of this invention.

The modified alumina support material used in this invention is prepared by first wetting an unmodified, conventional alumina support, as above described, with an aqueous solution of a salt of the required metal or metals; the so-wetted alumina then dried to remove water and then calcined for 5810 hours at an elevated temperature in the range of 300 to 600°C. during which the added metal salt is converted to metal oxide. An amount of metal salt is chosen so that the final modified alumina support contains from 0.5 to 3.0% by weight of incorporated metal.

# 0 039 723

The metal salt in the aqueous solution can be any desired soluble salt, such as a chloride, carbonate, of an alkali metal such as potassium, lithium, sodium, rubidium or cesium, preferably potassium or lithium, or of an alkaline earth metal such as calcium, strontium or barium, preferably the latter, or of a rare earth metal such as lanthanum or cerium or a mixture of rare earth metals such as the mixture called didymium which contains lanthanum and neodymium together with smaller amounts of praesodymium and samarium and even smaller amounts of other rare earth metals. Other mixtures of salts of the same or different metals recited, whether or not of the same class, may also be used; a mixture of a salt of an alkali metal, particularly potassium, with a salt of an alkaline earth metal, particularly barium, is especially desirable.

The modified alumina support having 0.5 to 3.0% of metal incorporated therein, prepared as above described, possesses physical characteristics as described for the conventional unmodified alumina support and can have copper catalyst, such as cuprous chloride or other cuprous or cupric salt, deposited thereon by the same techniques as used in formation of the known alumina-supported copper catalysts. The amount of copper deposited will depend on the activity desired and the specific fluidization characteristics of the support and can be as little as 2% by weight or as much as 10 to 12% by weight of the modified support material. The final catalyst composition containing the copper catalyst on the modified support is fluidizable like the support but certain specific characteristics, surface area and pore volume, for example, are, of course, modified by reason of the deposit of copper. The preferred catalyst compositions of this invention have a surface area in the range of 50 to 160 $M^2g$, some 15—20% lower than that of the support before deposit of copper.

The specific Examples set forth below further illustrate the nature of the catalyst composition of this invention, and especially their use to improve the fluid bed process for oxyhydrochlorination of ethylene to produce EDC. In each Example the catalyst composition is prepared using gamma alumina of the characteristics specified to prepare the modified support on which $CuCl_2$ is deposited and the fluid bed ethylene oxyhydrochlorination to form EDC is conducted in a bench scale fluid bed reactor of 22 mm. internal diameter and 24 inches heighth charged with 125 ml. of the fluid bed catalyst composition as described. The reactor is equipped with means for delivering the gaseous reactants— ethylene, oxygen (as air) and HCl—through the fluid bed reactor zone, for controlling the quantities of reactants and reaction conditions and for ascertaining from the effluent gases the conversion and yield of ethylene to EDC and carbon oxides.

Example I

This Example illustrates the preparation and use in ethylene oxyhydrochlorination to EDC of catalyst compositions consisting of copper deposited on fluidized alumina support modified by incorporation of an alkali metal.

Four catalyst compositions are prepared as follows: (A) gamma alumina is impregnated with 1% aqueous $K_2CO_3$ dried in a steam bath and calcined for 8—10 hours at 400°C. after which the K-modified alumina support is impregnated with 10% cupric chloride, dried slowly, calcined 8—10 hours at 275°C. and sieved to 80—325 mesh; (B) same as (A) except calcined after addition of $K_2CO_3$ and drying at 570°C.: (C) same as (A) except LiCl used in place of $K_2CO_3$ and (D) same as (A) except CsCl used in place of $K_2CO_3$.

Each catalyst composition is then separately charged to the fluid bed reactor where ethylene, oxygen and HCl in the molar ratios of 1.0 to 0.8 and 2.0 are reacted to form EDC under the conditions and with the yields and efficiencies as shown in Table I.

TABLE I

| Catalyst | Reaction temp. °C. | Reactants contact time seconds | % conv. $C_2H_4$ | % yield | | | % efficiency EDC |
|---|---|---|---|---|---|---|---|
| | | | | CO | $CO_2$ | EDC | |
| A | 220 | 13.1 | 98.1 | 0.21 | 0.58 | 99.0 | 97.1 |
| | 226 | 12.9 | 99.1 | 0.60 | 0.95 | 98.1 | 97.2 |
| | 220 | 14.4 | 98.6 | 0.62 | 0.82 | 98.4 | 97.0 |
| | 225 | 14.2 | 99.5 | 0.54 | 0.95 | 98.2 | 97.8 |
| | 221 | 16 | 99.5 | 0.59 | 1.03 | 98.1 | 97.6 |
| | 225 | 17.5 | 99.7 | 0.27 | 0.70 | 98.6 | 98.2 |
| | 220 | 21.1 | 100 | 0.59 | 1.21 | 97.9 | 97.9 |
| B | 221 | 10.4 | 97.4 | 0.27 | 0.42 | 99.1 | 96.7 |
| | 221 | 11.5 | 98.2 | 0.33 | 0.69 | 98.8 | 97.0 |
| | 225 | 11.4 | 98.2 | 0.26 | 0.54 | 98.9 | 97.1 |
| | 230 | 11.3 | 98.7 | 0.17 | 0.33 | 99.2 | 97.9 |
| | 235 | 11.2 | 100 | 0.59 | 0.83 | 98.0 | 98.0 |

4

TABLE I (contd.)

| Catalyst | Reaction temp. °C. | Reactants contact time seconds | % conv. $C_2H_4$ | % yield | | | % efficiency EDC |
|---|---|---|---|---|---|---|---|
| | | | | CO | $CO_2$ | EDC | |
| C | 220 | 11.0 | 98.6 | 0.15 | 1.19 | 98.3 | 97.0 |
| | 225 | 10.9 | 99.7 | 0.32 | 1.33 | 97.9 | 97.5 |
| | 220 | 13.2 | 99.5 | 0.18 | 1.37 | 98.1 | 97.6 |
| | 225 | 13.1 | 100 | 0.73 | 1.55 | 97.2 | 97.2 |
| | 220 | 15.8 | 99.7 | 0.71 | 1.42 | 97.4 | 97.2 |
| D | 220 | 12.7 | 98.8 | 0.5 | 1.03 | 98.3 | 96.8 |
| | 220 | 15.8 | 100 | 0.76 | 1.41 | 97.5 | 97.5 |
| | 220 | 20.0 | 100 | 0.81 | 2.07 | 96.6 | 96.6 |

It is noted from Table I that the EDC efficiency based on ethylene with each catalyst composition and under each reaction condition, is of the order of 97—98%. When, however, the same catalyst composition except for being prepared from the unmodified alumina support without first incorporating the alkali-metal therein is used in a "control" under the same reaction conditions, the maximum EDC efficiency based on ethylene is only of the order of 93—94%. Further, the fluid bed catalyst compositions used do not develop "stickiness" in use as occurs in another "control" when the alkali metal salt is deposited on the alumina support with the cuprous chloride catalyst.

Example II

This example illustrates catalyst compositions, and their use in fluid bed oxyhydrochlorination of ethylene to EDC, in which an alkaline earth metal is incorporated in the alumina support prior to deposit of $CuCl_2$ thereon. The catalyst composition is made by the procedure of Example I with the same materials except that 1% $Ba(OH)_2$ is used to prepare the modified support and the calcination temperature in its preparation is 570°C. This catalyst composition is used in the fluid bed oxyhydrochlorination of ethylene to EDC as described in Example I with a reaction temperature of 225°C. and a contact time of 10 seconds for a period of 14—18.5 hours. The ethylene conversions, EDC yield and EDC efficiency based on ethylene is shown in the following data:

| Elapsed time hours | % conversion $C_2H_4$ | % EDC | |
|---|---|---|---|
| | | Yield | Efficiency |
| 14 | 98.9 | 97.7 | 96.6 |
| 15.5 | 99.0 | 98.2 | 97.2 |
| 17.5 | 99.0 | 98.3 | 97.3 |
| 18.5 | 99.1 | 97.5 | 96.6 |

Example III

This example illustrates catalyst compositions prepared using rare earth metals and their use in ethylene oxyhydrochlorination. Catalyst compositions are prepared as in Example I but using lanthanum chloride ($LaCl_3$) and chlorides of rare earth metal mixture (RE $Cl_3$) respectively. They are used as in Example I in fluid bed oxyhydrochlorination of ethylene and reaction temperatures of 217°C. and 218°C., respectively; contact times of 11 and 17 seconds, respectively and molar ratios of $C_2H_4$ to $O_2$ to HCl of 1.0/.8/2.13 and 1.0/.8/2.02, respectively, with the following results:

| Metal salt | % conversion $C_2H_2$ | % EDC | |
|---|---|---|---|
| | | Yield | Efficiency |
| La $Cl_3$ | 99.3 | 98.3 | 97.6 |
| RE $Cl_3$ | 99.8 | 97.2 | 97.0 |

Example IV

A catalyst composition is prepared and used as in Example I except that a mixture of 1% KCl and 1% $BaCl_2$ is used to prepare the modified support on which $CuCl_2$ is deposited; the ratio of $C_2H_4$ to $O_2$ to HCl is 1/1.8/2.1 and the reaction is continued for a total of 282 hours. The data obtained are shown in Table II.

5

## 0 039 723

### TABLE II

| Reaction temp. °C. | Contact time seconds | Elapsed time hours | % conversion $C_2H_4$ | % EDC | |
|---|---|---|---|---|---|
| | | | | Yield | Efficiency |
| 225 | 10.5 | 6 | 98.2 | 99.8 | 98.3 |
| 225 | 10.5 | 27 | 98.3 | 99.4 | 97.8 |
| 220 | 12.7 | 48 | 100 | 99.2 | 99.2 |
| 218 | 12.7 | 168 | 98.7 | 99.0 | 97.7 |
| 218 | 11.1 | 222 | 96.6 | 99.6 | 96.3 |
| 220 | 11.0 | 233 | 96.9 | 99.1 | 96.1 |
| 215 | 13.7 | 236 | 96.8 | 99.6 | 96.4 |
| 220 | 13.6 | 279 | 98.5 | 99.2 | 97.7 |
| 218 | 17.3 | 282 | 99.3 | 99.0 | 98.4 |

Table II shows, in comparison to Table I, that yield of EDC from ethylene converted is above 99% even when the ratio of HCl in the reactants is increased and that efficiency of EDC is at 97—98% over a period of several days. This indicates that the catalyst composition of this Example is an excellent fluid bed catalyst for ethylene oxyhydrochlorination conducted with oxygen recycle since the relative proportion of carbon oxides in the vent gas to be recycled is reduced and excess HCl in the feed does not reduce EDC efficiency based on ethylene.

Although the Examples presented above all illustrate the use of the catalyst compositions of this invention in the fluid bed oxyhydrochlorination of ethylene to EDC and the advantages thereby achieved, it is to be understood that such catalyst compositions are also effective in the fluid bed oxyhydrochlorination of other gaseous hydrocarbons containing 1 to 4 carbon atoms such as methane, ethane, propylenes, propane, butylenes, butanes, etc. to produce other chlorinated hydrocarbons. They can be used advantageously wherever it is desired to have copper catalyst available on a fluidizable support.

### Claims

1. A catalyst composition comprising a copper catalyst deposited on a fluidizable gamma-alumina support, having a surface area of 60 to 200 square meters per gram, in which is incorporated prior to deposit of the copper catalyst from 0.5 to 3.0% by weight of at least one added metal selected from the class consisting of potassium, lithium, sodium, rubidium, cesium, alkaline earth metals and rare earth metals or combinations thereof.

2. The composition of claim 1 wherein the metal incorporated in the gamma-alumina support is an alkaline earth metal.

3. The composition of claim 1 wherein the metal incorporated in the gamma-alumina support is a rear earth metal.

4. The composition of claim 1 wherein the metal incorporated in the gamma-alumina support is potassium.

5. The composition of claim 1 wherein potassium and barium are incorporated in the gamma-alumina support.

6. In the process of oxyhydrochlorinating ethylene to produce 1,2-dichloroethane by contacting a vaporous mixture of ethylene, oxygen and hydrogen chloride with a fluidized catalyst composition disposed in a reaction zone and recovering 1,2-dichloroethane from the effluents of the reaction zone, the improvements in which the fluidized catalyst composition consists of 2 to 12% copper chloride supported on a fluidizable gamma-alumina having a surface area of 60 to 200 square meters per gram, in which is incorporated prior to deposit of copper thereon from 0.5 to 3.0% by weight of at least one added metal selected from the class consisting of potassium, lithium, sodium, rubidium, cesium, alkaline earth metals and rare earth metals or combinations thereof.

7. The process of claim 6 further characterized in that the added metal is potassium.

8. The process of claim 6 further characterized in that the fluidizable gamma-alumina support has incorporated therein, as added metals, both potassium and barium.

### Patentansprüche

1. Katalysator-Zusammensetzung, ethanltend einen Kupfer-Katalysator abgeschieden auf einem fluidisierbaren gamma-Aluminiumoxid-Träger mit einer spezifischen Oberfläche von 60 bis 200 m²/g, in den vor dem Abscheiden des Kupfer-Katalysators 0,5 bis 3 Gew.-% wenigstens eines zugesetzten Metalls ausgewählt aus der aus Kalium, Lithium, Natrium, Rubidium, Cäsium, Erdalkalimetallen und Seltenerdmetallen oder Kombinationen aus diesen bestehenden Gruppe eingearbeitet wurden.

6

2. Zusammensetzung nach Anspruch 1, dadurch gekennzeichnet, daß das in den gamma-Aluminiumoxid-Träger eingearbeitete Metall ein Erdalkalimetall ist.

3. Zusammensetzung nach Anspruch 1, dadurch gekennzeichnet, daß das in den gamma-Aluminiumoxid-Träger eingearbeitete Metall ein Seltenerdmetall ist.

4. Zusammensetzung nach Anspruch 1, dadurch gekennzeichnet, daß das in den gamma-Aluminiumoxid-Träger eingearbeitet Metall Kalium ist.

5. Zusammensetzung nach Anspruch 1, dadurch gekennzeichnet, daß Kalium und Barium in den gamma-Aluminiumoxid-Träger eingearbeitet sind.

6. Verfahren zur Oxyhydrochlorierung von Ethylene zur Herstellung von 1,2-Dichloroethan durch Kontaktieren eines dampfförmigen Gemischs aus Ethylen, Sauerstoff und Hydrogenchlorid mit einer in einer Reaktionszone angeordneten Wirbelschicht-Katalysator-Zusammensetzung und Gewinnung des 1,2-Dichloroethans aus dem Abstrom der Reaktionszone, dadurch gekennzeichnet, daß die Wirbelschicht-Katalysator-Zusammensetzung aus 2 bis 12% Kupferchlorid auf einem Träger aus fluidisierbarem gamma-Aluminiumoxid mit einer spezifischen Oberfläche von 60 bis 200 m²/g, in den vor dem Abscheiden des Kupfer-Katalysators 0,5 bis 3 Gew.% wenigstens eines zugesetzten Metalls ausgewählt aus der aus Kalium, Lithium, Natrium, Rubidium, Cäsium, Erdalkalimetallen und Seltenerdmetallen oder Kombinationen aus diesen bestehenden Gruppe eingearbeitet wurden, besteht.

7. Verfahren nach Anspruch 6, weiterhin dadurch gekennzeichnet, daß das zugesetzte Metall Kalium ist.

8. Verfahren nach Anspruch 6, weiterhin dadurch gekennzeichnet, daß in den fluidisierbaren gamma-Aluminiumoxid-Träger als zugesetzte Metalle sowohl Kalium als auch Barium eingearbeitet sind.

**Revendications**

1. Composition de catalyseur comprenant un catalyseur à base de cuivre déposé sur un support d'alumine gamma fluidisable, ayant une surface spécifique de 60 à 200 mètres carrés par gramme, auquel est incorporé, avant le dépôt du catalyseur à base de cuivre, de 0,5 à 3,0% en poids d'au moins un métal ajouté, choisi dans la classe consistant en le potassium, le lithium, le sodium, le rubidium, le césium, les métaux alcalino-terreux et les métaux des terres rares ou leurs combinaisons.

2. Composition selon la revendication 1, dans laquelle le métal incorporé au support d'alumine gamma est un métal alcalino-terreux.

3. Composition selon la revendication 1, dans laquelle le métal incorporé au support d'lumine gamma est un métal des terres rares.

4. Composition selon la revendication 1, dans laquelle le métal incorporé au support d'alumine gamma est le potassium.

5. Composition selon la revendication 1, dans laquelle on incorpore du potassium et du baryum au support d'alumine gamma.

6. Dans le procédé d'oxyhydrochloruration de l'éthylène pour produire du dichloro-1,2 éthane par mise en contact d'un mélange, sous forme vapeur, d'éthylène, d'oxygène et de chlorure d'hydrogène avec une composition de catalyseur fluidisée disposée dans un zone de réaction et récupération du dichloro-1,2 éthane à partir des effluents de la zone de réaction, les améliorations selon lesquelles la composition de catalyseur fluidisée consiste en 2 à 12% de chlorure de cuivre sur un support d'alumine gamma fluidisable ayant une surface spécifique de 60 à 200 mètres carrés par gramme, auquel est incorporé, avant le dépôt du cuivre, de 0,5 à 3,0% en poids d'au moins un métal ajouté choisi dans la classe consistant en le potassium, le lithium, le sodium, le rubidium, le césium, les métaux alcalino-terreux et les métaux des terres rares ou leurs combinaisons.

7. Procédé selon la revendication 6, caractérisé en outre en ce que le métal ajouté est le potassium.

8. Procédé selon la revendication 6, caractérisé en outre en ce que le support d'alumine gamma fluidisable comporte incorporés, à titre de métaux ajoutés, à la fois du potassium et du baryum.